# FASCICULE DE BREVET EUROPEEN

(11) **EP 0 571 303 B1**
(45) Date de publication et mention de la délivrance du brevet: **20.08.1997**
(21) Numéro de dépôt: 93420196.3
(22) Date de dépôt: 14.05.1993
(51) Int. Cl.: A61M 1/16

(54) **Circuit hydraulique pour rein artificiel avec dispositif de filtration du liquide de dialyse**
Hydraulischer Kreislauf für eine künstliche Niere mit einer Filtereinrichtung für die Dialyseflüssigkeit
Hydraulic circuit for an artificial kidney with a filtration device for the dialysis liquid

(30) Priorité: 19.05.1992 FR 9206329
(43) Date de publication de la demande: 24.11.1993
(73) Titulaire: HOSPAL AG, 4008 Basel (CH)
(72) Inventeur: Tusini, Andrea, I-41100 Modena (IT); Vinci, Luca, I-46025 Poggio Rusco, Mandova (IT)

(56) Documents cités:
- EP-A- 0 058 304
- EP-A- 0 276 376
- DE-U- 8 305 713
- US-A- 4 773 991
- US-A- 4 784 763

## Description

La présente invention est relative au domaine du traitement extracorporel du sang par dialyse et/ ou par ultrafiltration.

Plus particulièrement, l'invention concerne un circuit hydraulique pour rein artificiel dans lequel le circuit de liquide de dialyse comporte un ultrafiltre destiné à débarrasser le liquide de dialyse des microorganismes et éventuellement des éléments pyrogènes qu'il pourrait contenir.

Avec le développement des hémodialyseurs à haute perméabilité, on a vu s'accroître l'intérêt pour l'utilisation d'un liquide de dialyse ayant de bonnes qualités biologiques. En effet, bien que, pour purifier le sang à traiter, on souhaite que le sens des échanges dans l'hémodialyeur soit du compartiment sang vers le compartiment liquide de dialyse, il n'est pas exclu qu'il y ait à l'inverse des transferts du liquide de dialyse vers le sang, notamment par diffusion ou même par convection dans le cas où la pression dans le compartiment liquide de dialyse deviendrait supérieure à la pression existant dans le compartiment sang.

Aussi, le brevet US 4 834 888 propose un rein artificiel comportant un filtre pour stériliser en ligne le liquide de dialyse avant son entrée dans l'hémodialyseur. Selon ce document, le filtre utilisé pour stériliser le liquide de dialyse comporte deux compartiments séparés par une membrane capable de retenir les germes. Le liquide provenant d'une source de liquide de dialyse est filtré en totalité à travers ce filtre avant de pénétrer dans l'hémodialyseur. Afin d'éviter le colmatage du filtre, il est prévu selon ce document, de rincer à la fin de chaque séance d'hémodialyse ainsi qu'éventuellement en cours de séance, le compartiment relié à la source de liquide de dialyse.

A cette fin, ce compartiment possède une sortie reliée à la canalisation d'évacuation du liquide de dialyse usagé provenant de l'hémodialyseur. Lors de la phase de rinçage du filtre, l'opération de stérilisation du liquide par filtration est arrêtée ; le liquide pénétrant dans le filtre balaie alors la surface de la membrane afin de décrocher les germes et éléments pyrogènes qui s'y sont déposés, puis rejoint directement la canalisation d'évacuation du liquide de dialyse usagé.

Afin de vérifier l'intégrité de la membrane de ce filtre de stérilisation, ce document décrit un test particulier réalisé entre deux séances de dialyse. Ce test implique l'introduction d'air à l'intérieur d'une partie au moins du circuit de liquide de dialyse et ne peut donc être effectué que lorsque l'hémodialyseur est déconnecté de l'appareil de dialyse.

Ainsi, le dispositif décrit dans ce document présente l'inconvénient de ne pas permettre de vérifier, en cours d'utilisation, l'intégrité et l'efficacité de la membrane du filtre.

La présente invention a pour objet un circuit hydraulique pour rein artificiel permettant de vérifier en permanence le bon état de l'ultrafiltre sans arrêter son fonctionnement.

La présente invention a également pour objet un circuit hydraulique pour rein artificiel permettant à son utilisateur d'être averti en cas de colmatage ou de rupture de la membrane filtrant le liquide de dialyse.

A cette fin, la présente invention propose un circuit hydraulique pour un rein artificiel comportant:
- une canalisation d'alimentation en liquide de dialyse ayant une première portion ayant une extrémité connectée à une source de liquide de dialyse et une seconde portion ayant une extrémité connectable à un hémodialyseur ou à un circuit pour circulation extracorporelle de sang;
- un ultrafiltre ayant une première et une seconde chambre séparées par une membrane apte à retenir des éléments microbiens et pyrogènes, la première portion de la canalisation d'alimentation ayant son autre extrémité connectée à une entrée de la première chambre et la seconde portion de la canalisation d'alimentation ayant son autre extrémité connectée à une sortie de la seconde chambre,
   caractérisé en ce qu'il comporte :
   - des moyens pour déterminer la pression transmembranaire dans l'ultrafiltre ;
   - des moyens de dérivation pour relier directement la première et la seconde portions de la canalisation d'alimentation et pour interdire le passage de liquide dans l'ultrafiltre ;
   - des moyens de commande pour comparer la pression transmembranaire mesurée à au moins une valeur de seuil et pour commander les moyens de dérivation, lorsque la valeur mesurée atteint la valeur de seuil, de façon que la première et la seconde portions de la canalisation d'alimentation soit reliées directement et que le passage de liquide soit interdit dans l'ultrafiltre.

Ceci est particulièrement avantageux lorsque le liquide filtré est utilisé comme liquide de dialyse car, dans le cas où on détecte un défaut d'intégrité de la membrane, il n'est pas obligatoire d'arrêter le traitement mais il est possible de poursuivre la séance sans cependant faire courir au patient de risques graves.

Avantageusement, la première chambre de l'ultrafiltre comporte une sortie reliée à une canalisation d'évacuation pour permettre un balayage tangentiel de la membrane du filtre.

Ainsi, le dépôt de microorganismes ou d'éléments pyrogènes et par conséquent le colmatage de la membrane sont réduits au maximum ; ceci permet d'augmenter la durée de vie de l'ultrafiltre et de diminuer le risque de passage d'éléments indésirables dans le liquide pénétrant dans l'hémodialyseur ou dans le circuit extracorporel de sang, pour le cas où la membrane de l'ultrafiltre viendrait à être endommagée.

Selon un mode de réalisation particulier, le circuit hydraulique de l'invention comporte des moyens pour régler le débit de liquide dans la canalisation d'alimentation en aval de l'ultrafiltre.

Ainsi, quelle que soit la perte de charge provoquée dans le circuit de liquide de dialyse par la présence d'un ultrafiltre, le débit de liquide de dialyse pénétrant dans l'hémodialyseur ou dans le circuit extracorporel de sang peut être maintenu constant.

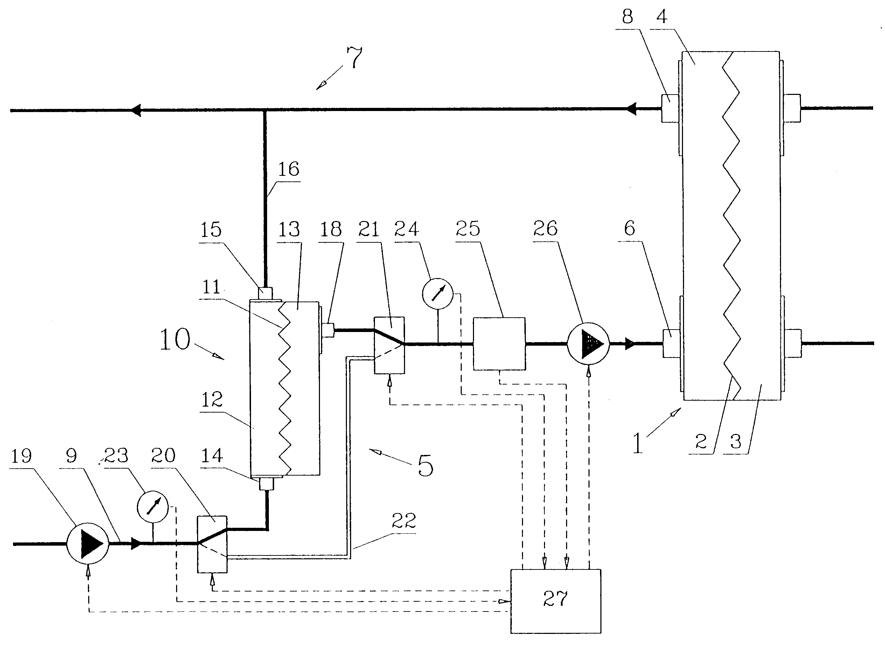

D'autres particularités et avantages apparaitront au cours de la description qui va suivre, en référence au dessin qui illustre, de façon schématique, un mode particulier de réalisation de la présente invention, dans son application à l'utilisation du liquide filtré comme liquide de dialyse.

Sur la figure, on représente schématiquement une partie d'un rein artificiel (seuls les éléments relatifs à l'invention ont été représentés) qui comporte de façon classique un hémodialyseur 1 séparé par une membrane 2 en deux compartiments 3 et 4. Le compartiment 3 est parcouru par le sang à traiter alors que le compartiment 4 est parcouru par du liquide de dialyse destiné à l'épuration du sang. Le circuit pour la circulation du liquide de dialyse à l'intérieur du rein artificiel comporte une portion amont 5 destinée au liquide de dialyse frais pénétrant dans l'hémodialyseur par une entrée 6, ainsi qu'une portion aval 7 destinée au liquide de dialyse usagé quittant l'hémodialyseur par une sortie 8. La portion amont 5 du circuit de liquide de dialyse est constituée par une canalisation principale 9 apte à conduire le liquide de dialyse frais d'une source (non représentée) vers l'hémodialyseur. Cette canalisation 9 comporte un ultrafiltre 10 possédant une membrane d'ultrafiltration 11 apte à retenir les microorganismes et les éléments pyrogènes du liquide de dialyse.

Cette membrane 11 sépare donc l'ultrafiltre 10 en deux compartiments :
- un compartiment 12 recevant le liquide de dialyse frais éventuellement contaminé,
- un compartiment 13 recueillant le liquide de dialyse ultrafiltré.

Le compartiment 12 est muni de deux ouvertures : une ouverture 14 d'entrée du liquide de dialyse à filtrer, reliée à la canalisation 9, ainsi qu'une ouverture 15 de sortie du liquide de dialyse non filtré reliée à une canalisation 16, elle-même reliée à la portion aval 7 du circuit de liquide de dialyse.

Le compartiment 13 de l'ultrafiltre 10 est pourvu d'une ouverture 18 de sortie du liquide filtré.

La canalisation 9 comporte également, en amont de l'ultrafiltre 10, une pompe 19 de circulation du liquide de dialyse.

Selon l'invention, on dispose sur la canalisation 9, en amont de l'ultrafiltre et en aval de la pompe 19, un capteur de pression 23 qui permet de mesurer en continu la pression du liquide de dialyse en amont de l'ultrafiltre.

De même, un capteur de pression 24 situé entre l'ultrafiltre 10 et l'hémodialyseur 1 permet la mesure en continu de la pression du liquide de dialyse filtré à la sortie de l'ultrafiltre.

Avantageusement, on dispose de part et d'autre de l'ultrafiltre 10 une vanne trois voies : une vanne 20 est située en amont de l'ultrafiltre 10 mais en aval du capteur de pression 23, alors qu'une vanne 21 est située en aval de l'ultrafiltre mais en amont du capteur de pression 24. Ces vannes 20 et 21 sont directement reliées par une canalisation de dérivation 22.

On dispose en outre, en aval du capteur de pression 24 un débitmètre 25 ainsi qu'une pompe de circulation 26.

Une unité de contrôle 27 reçoit les informations provenant des capteurs de pression 23 et 24 ainsi que du débitmètre 25, et agit sur le fonctionnement des vannes 20, 21 et des pompes 19 et 26.

Le fonctionnement du rein artificiel ainsi décrit est le suivant.

Le liquide de dialyse est mis en circulation grâce à l'action de la pompe 19 dont le débit est fixé par exemple à 0,54 l/min. Les vannes 20 et 21 étant positionnées de manière que la circulation du liquide se fasse par l'ultrafiltre 10 et non par la canalisation 22, le liquide de dialyse s'écoule dans la canalisation 9, puis pénètre dans l'ultrafiltre 10.

Grâce à l'action de la pompe 26 dont le débit est fixé par exemple à 0,5 l/min, une fraction importante du liquide pénétrant dans l'ultrafiltre est filtrée par passage au travers de la membrane 11 et pénètre dans l'hémodialyseur par l'entrée 6 puis ressort par l'ouverture 8 pour être conduite, grâce à la portion aval du circuit de liquide de dialyse vers des moyens d'évacuation ou de régénération (non représentés).

Selon une caractéristique de l'invention, les valeurs de pression mesurées par les capteurs 23 et 24 et correspondant aux valeurs de pression du liquide de part et d'autre de la membrane d'ultrafiltration 11 sont transmises en cours de fonctionnement de l'ultrafiltre à l'unité de contrôle 27 qui détermine ainsi la valeur de la pression transmembranaire. Cette valeur de pression transmembranaire (PTM) est comparée à une ou plusieurs valeurs de seuil préalablement enregistrées par l'opérateur dans l'unité de contrôle en fonction de l'ultrafiltre utilisé. Ainsi, il est possible de fixer une valeur haute de seuil correspondant à un colmatage de la membrane 11 et une valeur basse de seuil correspondant à un endommagement de la membrane tel qu'une rupture. Si la valeur de PTM calculée atteint l'une ou l'autre des valeurs de seuil, l'unité de contrôle 27 commande l'émission d'un signal destiné à avertir l'opérateur, grâce par exemple au déclenchement d'une alarme visuelle et/ou accoustique ou par l'émission d'un message adéquat sur un écran (non représenté) servant d'interface entre l'opérateur et la machine. L'opérateur peut alors agir manuellement sur des moyens de commande des vannes 20 et 21 pour les manoeuvrer de manière que le liquide de dialyse ne s'écoule plus à travers l'ultrafiltre 10 mais emprunte la canalisation de dérivation 22. Ainsi, la séance de traitement du sang peut être poursuivie avec du liquide de dialyse non filtré jusqu'à ce que l'opérateur ait procédé au remplacement du filtre.

Alternativement, l'unité de contrôle 27 peut commander directement la manoeuvre des vannes 20 et 21 lorsque la pression transmembranaire calculée atteint l'une ou l'autre des valeurs de seuil.

Selon une caractéristique avantageuse de l'invention, une fraction du liquide de dialyse pénétrant dans l'ultrafiltre 10 ne traverse pas la membrane 11 mais ressort directement par l'ouverture 15 pour rejoindre, par l'intermédiaire de la canalisation 16, le liquide de dialyse usagé provenant de l'hémodialyseur. Ce flux tangentiel continu est obtenu grâce à la différence existant entre le débit fourni par la pompe 19 et celui de la pompe 26. Alternativement, il est possible de munir la canalisation 16 d'une pompe bas débit (non représentée), ce qui permet de contrôler précisément le débit du flux tangentiel. Le balayage permanent de la membrane d'ultrafiltration par un flux tangentiel de liquide a pour avantage d'entraîner avec lui les microorganismes qui se déposent moins sur la membrane ; ceci augmente la durée de vie de l'ultrafiltre et réduit les risques d'entraînement d'un passage important d'éléments microbiens et/ou pyrogènes dans le liquide entrant dans l'hémodialyseur, en cas de rupture de la membrane d'ultrafiltration.

Selon une autre caractéristique de l'invention, le flux de liquide de dialyse filtré au travers de la membrane 11 est contrôlé très précisément, grâce par exemple à l'utilisation d'une pompe 26 à débit très précis ou par l'adjonction d'un débitmètre 25 dont les informations sont transmises à l'unité de contrôle 27 qui commande en conséquence le fonctionnement de la pompe 26. Cet asservissement de la pompe 26 au débit mesuré par le débitmètre 25 permet de maintenir constant le débit de liquide de dialyse pénétrant dans l'hémodialyseur 1.

De nombreuses variantes de réalisation sont à la portée de l'homme de métier sans sortir du cadre de la présente invention.

Ainsi, il est possible de prévoir pour la pression transmembranaire plusieurs valeurs de seuil haut et bas correspondant à différents degrés de colmatage ou d'endommagement de la membrane d'ultrafiltration et nécessitant des actions différentes de la part de l'opérateur ou de la machine.

De même, il est possible d'améliorer la précision du débit de liquide fourni par la pompe de circulation 19 en ajoutant sur la canalisation 9 un débitmètre (non représenté) auquel le fonctionnement de la pompe 19 serait asservi.

La présente invention a été décrite dans son application particulière au cas où le liquide filtré est utilisé comme liquide de dialyse. 11 est également possible de l'appliquer au cas où le liquide de dialyse filtré est injecté, en tant que liquide de substitution, au circuit extracorporel de sang. Ceci peut être le cas en hémofiltration où l'épuration du sang est effectuée uniquement par ultrafiltration sans circulation de liquide de dialyse de l'autre côté de la membrane au contact de laquelle circule le sang, ou également en hémodiafiltration où le sang est épuré à la fois par dialyse et par ultrafiltration avec réinjection dans le circuit extracorporel de sang d'un liquide de substitution.

## Revendications

1. Circuit hydraulique pour un rein artificiel comportant:
- une canalisation d'alimentation (9) en liquide de dialyse ayant une première portion ayant une extrémité connectée à une source de liquide de dialyse et une seconde portion ayant une extrémité connectable à un hémodialyseur (1) ou à un circuit pour circulation extracorporelle de sang;
- un ultrafiltre (10) ayant une première et une seconde chambre (12, 13) séparées par une membrane (11) apte à retenir des éléments microbiens et pyrogènes, la première portion de la canalisation d'alimentation (9) ayant son autre extrémité connectée à une entrée (14) de la première chambre (12) et la seconde portion de la canalisation d'alimentation (9) ayant son autre extrémité connectée à une sortie (18) de la seconde chambre (13),
caractérisé en ce qu'il comporte :
- des moyens (23, 24, 27) pour déterminer la pression transmembranaire dans l'ultrafiltre (10) ;
- des moyens de dérivation (20, 21, 22) pour relier directement la première et la seconde portions de la canalisation d'alimentation (9) et pour interdire le passage de liquide dans l'ultrafiltre (10) ;
- des moyens de commande (27) pour comparer la pression transmembranaire mesurée à au moins une valeur de seuil et pour commander les moyens de dérivation (20, 21, 22), lorsque la valeur mesurée atteint la valeur de seuil, de façon que la première et la seconde portions de la canalisation d'alimentation (9) soit reliées directement et que le passage de liquide soit interdit dans l'ultrafiltre (10).

2. Circuit selon la revendication 1, caractérisé en ce que les moyens commande (27) sont prévus pour comparer la pression transmembranaire mesurée à une valeur de seuil haut correspondant à un colmatage de la membrane (11) de l'ultrafiltre (10) à une valeur de seuil de seuil bas correspondant à une rupture de la membrane (11) de l'ultrafiltre (10).

3. Circuit selon une des revendications 1 et 2, caractérisé en ce que les moyens commande (27) sont prévus pour déclencher une alarme visuelle ou acoustique lorsque la valeur mesurée atteint la valeur de seuil.

4. Circuit selon une des revendications 1 à 3, caractérisé en ce que la première chambre (12) de l'ultrafiltre (10) comporte une sortie (15) reliée à une canalisation d'évacuation (16) pour permettre un balayage tangentiel de la membrane (11) du filtre (10).

5. Circuit selon une des revendications 1 à 4, caractérisé en ce qu'il comporte des moyens (19, 26, 27) pour régler le débit du liquide sortant de la première chambre (12) de l'ultrafiltre (10) par la sortie (15) reliée à la canalisation d'évacuation (16).

6. Circuit selon la revendication 5, caractérisé en ce que les moyens pour régler le débit du liquide sortant de la première chambre (12) de l'ultrafiltre (10) comprennent:
- une première pompe (19) disposée sur la première portion de la canalisation d'alimentation (9);
- une deuxième pompe (26) disposée sur la deuxième portion de la canalisation d'alimentation (9).

7. Circuit selon une des revendications 1 à 6, caractérisé en ce qu'il comporte des moyens (25, 26, 27) pour régler le débit de liquide dans la canalisation d'alimentation (9) en aval de l'ultrafiltre (10).

8. Circuit selon la revendication 7, caractérisé en ce que les moyens pour régler le débit de liquide dans la canalisation d'alimentation (9) en aval de l'ultrafiltre (10) comprennent une pompe (26) et un débitmètre (25) disposés sur la seconde portion de la canalisation d'alimentation (9), et des moyens (27) pour commander la pompe (26) en fonction des informations délivrées par le débitmètre (27).

9. Circuit selon une des revendications 1 à 8, caractérisé en ce que les moyens pour déterminer la pression transmembranaire dans l'ultrafiltre (10) comprennent un premier et un second capteurs de pression (23, 24) disposés respectivement sur la première et sur la seconde portions de la canalisation d'alimentation (9), la valeur de la pression transmembranaire étant déterminée à partir des informations fournies par les capteurs de pression.

10. Circuit selon une des revendications 1 à 9, caractérisé en ce que les moyens de dérivation comprennent une canalisation de dérivation (22) ayant une extrémité connectée, par l'intermédiaire d'une vanne trois voies (20) à la première portion de la canalisation d'alimentation (9), et une autre extrémité connectée, par l'intermédiaire d'une vanne trois voies (21) à la seconde portion de la canalisation d'alimentation (9).

## Claims

1. Hydraulic circuit for an artificial kidney comprising :
- a supply pipe (9) for dialysis liquid having a first portion with an end connected to a dialysis liquid source and a second portion with an end which can be connected to a hemodialyzer (1) or to a circuit for extracorporeal blood circulation ;
- an ultrafilter (10) having a first and a second chamber (12, 13) separated by a membrane (11) capable of retaining microbial and pyrogenic elements, the first portion of the supply pipe (9) having its other end connected to an inlet (14) of the first chamber (12) and the second portion of the supply pipe (9) having its other end connected to an outlet (18) of the second chamber (13),
characterized in that it comprises :
- means (23, 24, 27) for determining the transmembrane pressure in the ultrafilter (10) ;
- by-pass means (20, 21, 22) for directly linking the first and the second portions of the supply pipe (9) and for preventing the liquid from flowing in the ultrafilter (10) ;
- control means (27) for comparing the measured transmembrane pressure with at least one threshold value and for controlling the by-pass means (20, 21, 22), when the measured value reaches the threshold value, so that the first and the second portions of the supply pipe (9) are directly linked and so that the liquid cannot flow in the ultrafilter (10).

2. Circuit according to claim 1, characterized in that the control means (27) is designed to compare the measured transmembrane pressure with a high threshold value corresponding to a clogging of the membrane (11) of the ultrafilter, and with a low threshold value corresponding to a breakage of the membrane (11) of the ultrafilter (10).

3. Circuit according to one of claims 1 and 2, characterized in that the control unit (27) is designed to trigger off a visual or acoustic signal when the measured value reaches the threshold value.

4. Circuit according to one of claims 1 to 3, characterized in that the first chamber (12) of the ultrafilter (10) comprises an outlet (15) connected to a discharge line (16) in order to allow a tangential flow of liquid along the membrane (11) of the ultrafilter (10).

5. Circuit according to one of claims 1 to 4, characterized in that it comprises means (19, 26, 27) for controlling the flow rate of the liquid flowing out of the first chamber (12) of the ultrafilter (10) through the outlet (15) connected to the draining pipe (16).

6. Circuit according to claim 5, characterized in that the means for controlling the flow rate of the liquid flowing out of the first chamber (12) of the ultrafilter (10) comprises :
- a first pump (19) arranged on the first portion of the supply pipe (9);
- a second pump (26) arranged on the second portion of the supply pipe (9).

7. Circuit according to one of claims 1 to 6, characterized in that it comprises means (25, 26, 27) for controlling the flow rate of the liquid in the supply pipe (9) upstream of the ultrafilter (10).

8. Circuit according to claim 7, characterized in that the means for controlling the flow rate of the liquid in the supply pipe (9) upstream of the ultrafilter (10) comprises a pump (26) and a flowmeter (25) located on the second portion of the supply line (9), and means (27) for controlling the pump (26) in accordance with the information given by the flowmeter (27).

9. Circuit according to one of claims 1 to 8, characterized in that the means for determining the transmembrane pressure in the ultrafilter (10) comprises a first and a second pressure sensors (23, 24) respectively located on the first and the second portion of the supply pipe (9), the value of the transmembrane pressure being determined from the information given by the pressure sensors.

10. Circuit according to one of claims 1 to 9, characterized in that the by-pass means comprises a branch pipe (22) having an end connected to the first portion of the supply line (9) through a three-way valve (20) and another end connected to the second portion of the supply line (9) through a three-way valve (21).

## Patentansprüche

1. Hydraulikkreislauf für eine künstliche Niere mit folgendem:
- einer Dialyseflüssigkeitszufuhrleitung (9) mit einem ersten Teil, bei dem ein Ende mit einer Dialyseflüssigkeitsquelle verbunden ist, und einem zweiten Teil, bei dem ein Ende mit einem Hämodialysator (1) oder einem Kreislauf zum extrakorporalen Umlauf von Blut verbunden werden kann;
- einem Ultrafilter (10) mit einer ersten und einer zweiten Kammer (12, 13), die durch eine zum Zurückhalten von Mikroben und Pyrogenen geeignete Membran (11) getrennt sind, wobei das andere Ende des ersten Teils der Zufuhrleitung (9) mit einem Eingang (14) der ersten Kammer (12) verbunden ist und das andere Ende des zweiten Teils der Zufuhrleitung (9) mit einem Ausgang (18) der zweiten Kammer (13) verbunden ist,
dadurch gekennzeichnet, daß er folgendes umfaßt:
- Mittel (23, 24, 27) zur Bestimmung des Transmembrandrucks im Ultrafilter (10);
- Umleitungsmittel (20, 21, 22) zur direkten Verbindung des ersten und zweiten Teils der Zufuhrleitung (9) und zur Sperrung des Ultrafilters (10) für Flüssigkeit;
- Steuermittel (27) zum Vergleichen des gemessenen Transmembrandrucks mit mindestens einem Schwellenwert und zum Steuern der Umleitungsmittel (20, 21, 22) derart, daß der erste und zweite Teil der Zufuhrleitung (9) direkt miteinander verbunden werden und das Ultrafilter (10) für Flüssigkeit gesperrt wird, wenn der gemessene Wert den Schwellenwert erreicht.

2. Kreislauf nach Anspruch 1, dadurch gekennzeichnet, daß die Steuermittel (27) vorgesehen sind, um den gemessenen Transmembrandruck mit einem einer Verstopfung der Membran (11) des Ultrafilters (10) entsprechenden oberen Schwellenwert und mit einem einem Riß der Membran (11) des Ultrafilters (10) entsprechenden unteren Schwellenwert zu vergleichen.

3. Kreislauf nach Anspruch 1 oder 2, dadurch gekennzeichnet, daß die Steuermittel (27) vorgesehen sind, um einen visuellen oder akustischen Alarm auszulösen, wenn der gemessene Wert den Schwellenwert erreicht.

4. Kreislauf nach einem der Ansprüche 1 bis 3, dadurch gekennzeichnet, daß die erste Kammer (12) des Ultrafilters (10) einen mit einer Abflußleitung (16) verbundenen Ausgang (15) umfaßt, so da die Membran (11) des Filters (10) tangentiell gespült werden kann.

5. Kreislauf nach einem der Ansprüche 1 bis 4, dadurch gekennzeichnet, daß er Mittel (19, 26, 27) umfaßt, die den Durchfluß der über den mit der Abflußleitung (16) verbundenen Ausgang (15) aus der ersten Kammer (12) des Ultrafilters (10) austretenden Flüssigkeit regulieren.

6. Kreislauf nach Anspruch 5, dadurch gekennzeichnet, daß die Mittel zur Regulierung des Durchflusses der aus der ersten Kammer (12) des Ultrafilters (10) austretenden Flüssigkeit folgendes umfassen:
- eine am ersten Teil der Zufuhrleitung (9) angeordnete Pumpe (19) und
- eine am zweiten Teil der Zufuhrleitung (9) angeordnete Pumpe (26).

7. Kreislauf nach einem der Ansprüche 1 bis 6, dadurch gekennzeichnet, daß er Mittel (25, 26, 27) zur Regulierung des Flüssigkeitsdurchflusses in der dem Ultrafilter (10) nachgeschalteten Zufuhrleitung (9) umfaßt.

8. Kreislauf nach Anspruch 7, dadurch gekennzeichnet, daß die Mittel zur Regulierung des Flüssigkeitsdurchflusses in der dem Ultrafilter (10) nachgeschalteten Zufuhrleitung (9) eine Pumpe (26) und einen Durchflußmesser (25), die am zweiten Teil der Zufuhrleitung (9) angeordnet sind, und Mittel (27) zur Steuerung der Pumpe (26) in Abhängigkeit von den vom Durchflußmesser (25) gelieferten Informationen umfassen.

9. Kreislauf nach einem der Ansprüche 1 bis 8, dadurch gekennzeichnet, daß die Mittel zur Bestimmung des Transmembrandrucks im Ultrafilter (10) einen ersten und zweiten Druckaufnehmer (23, 24) umfassen, die am ersten bzw. zweiten Teil der Zufuhrleitung (9) angeordnet sind, wobei der Transmembrandruckwert aus von den Druckaufnehmern gelieferten Informationen bestimmt wird.

10. Kreislauf nach einem der Ansprüche 1 bis 9, dadurch gekennzeichnet, daß die Umleitungsmittel eine Umleitung (22) umfassen, deren eines Ende über ein Dreiwegeventil (20) mit dem ersten Teil der Zufuhrleitung (9) und deren anderes Ende über ein Dreiwegeventil (21) mit dem zweiten Teil der Zufuhrleitung (9) verbunden ist.
